# EUROPEAN PATENT APPLICATION

(11) **EP 4 052 704 A1**
(43) Date of publication of application: **07.09.2022**
(21) Application number: 20883124.8
(22) Date of filing: 07.10.2020
(51) Int. Cl.: A61K 31/4164, A23L 33/175, A61P 25/20, C07D 233/84

(54) **COMPOSITION FOR SLEEP IMPROVEMENT COMPRISING ERGOTHIONEINE OR SALT THEREOF**

(30) Priority: 28.10.2019 JP 2019195440
(71) Applicant: Suntory Holdings Limited, Osaka 530-8203 (JP)
(72) Inventor: KATSUBE, Makoto, Soraku-gun, Kyoto 619-0284 (JP); WATANABE, Hiroshi, Soraku-gun, Kyoto 619-0284 (JP); WAKABAYASHI, Kenichi, Soraku-gun, Kyoto 619-0284 (JP); MURAYAMA, Norihito, Soraku-gun, Kyoto 619-0284 (JP); SUZUKI, Kosuke, Soraku-gun, Kyoto 619-0284 (JP); TATEBAYASHI, Yoshitaka, Tokyo 156-8506 (JP); MATSUDA, Yoshiki, Tokyo 156-8506 (JP); OZAWA, Nobuyuki, Tokyo 156-8506 (JP); SHINOZAKI, Takiko, Tokyo 156-8506 (JP)
(74) Representative: Vossius & Partner Patentanwälte Rechtsanwälte mbB
(86) International application number: PCT/JP2020/037973
(87) International publication number: WO 2021/085062

(57) **Abstract**

The present invention aims to provide a composition for sleep improvement, which contains a highly safe substance as an active ingredient, and a method of improving sleep. The present invention relates to, for example, a composition for sleep improvement, which contains L-ergothioneine or a salt thereof as an active ingredient.

## Description

### TECHNICAL FIELD

The present invention relates to a composition for sleep improvement. The present invention also relates to a method of improving sleep and the like.

### BACKGROUND ART

Sleep is an important physiological activity that occupies about one third of the lifespan. Recent years witness an increasing number of people dissatisfied with sleep quality due to life rhythm disturbance, stress, or aging, for example. A reduction in sleep quality involves symptoms such as a longer sleep latency (having trouble with falling asleep), early morning awakening (waking up early in the morning), wake after sleep onset (frequent nocturnal awakenings), and deep sleep failure (not feeling well asleep or feeling shallow sleep).

Sleeping pills are used for treatment to improve sleep quality. However, sleeping pills have side effects and some people have anxiety about taking sleeping pills, for example, so that food-derived substances that can improve sleep quality have been developed. For example, Patent Literature 1 discloses an oral sleeping improver containing at least one selected from the group consisting of menthol and a glycoside thereof as an active ingredient.

Ergothioneine is an amino acid found in mushrooms and the like. L-ergothioneine is present in nature. Patent Literature 2 discloses an agent that promotes production and/or secretion of an antimicrobial substance in paneth cells, the agent containing ergothioneine extracted from mushrooms as an active ingredient.

### CITATION LIST

### - Patent Literature

Patent Literature 1: JP 2019-6717 A
Patent Literature 2: JP 2012-180329 A

### SUMMARY OF INVENTION

### - Technical Problem

Patent Literatures 1 and 2 nowhere state that L-ergothioneine or a salt thereof is effective in improving sleep.

The present invention aims to provide a composition for sleep improvement, the composition containing a highly safe substance as an active ingredient. The present invention also aims to provide a method of improving sleep.

### - Solution to Problem

As a result of extensive studies to solve the above issue, the present inventors found that L-ergothioneine has a sleep improving effect.

Specifically, the present invention relates to the following composition for sleep improvement, for example.
(1) A composition for sleep improvement, the composition containing L-ergothioneine or a salt thereof as an active ingredient.
(2) The composition for sleep improvement according to (1) above, wherein the sleep improvement is at least one selected from the group consisting of reduction in sleep latency, reduction in duration and number of wake after sleep onset, and increase in ratio of NREM sleep duration to total sleep time.
(3) The composition for sleep improvement according to (1) or (2) above, wherein the composition is an oral composition.
(4) The composition for sleep improvement according to any one of (1) to (3) above, wherein the composition is a food or beverage.
(5) A method of improving sleep, the method including administering L-ergothioneine or a salt thereof.
(6) Use of L-ergothioneine or a salt thereof for improving sleep.

### - Advantageous Effects of Invention

The present invention provides a composition for sleep improvement, the composition containing a highly safe substance as an active ingredient. The present invention can also provide a method of improving sleep.

### BRIEF DESCRIPTION OF DRAWINGS

Fig. 1 is a graph showing the duration of episode of each sleep stage (NREM sleep or REM sleep) and sleep duration in a control group (stress (-)), a non-administration group (stress (+), ergothioneine administration (-)), and an ergothioneine administration group (stress (+), ergothioneine administration (+)) during the light phase.
Fig. 2 is a graph showing results of evaluation of the effect of L-ergothioneine on the number of sleep fragmentation episodes in each sleep stage and the total number of sleep fragmentation episodes during the light phase.
Fig. 3 is a graph showing results of evaluation of the latency from the start of the light phase to the first episode of NREM sleep in the control group (stress (-)), the non-administration group (stress (+), ergothioneine administration (-)), and the ergothioneine administration group (stress (+), ergothioneine administration (+)).
Fig. 4 is a graph showing the ratio of NREM sleep stage 1 (N1) to total sleep time in the control food group and the test food group.
Fig. 5 is a graph showing the ratio of NREM sleep stage 2 (N2) to total sleep time in the control food group and the test food group.
Fig. 6 is a graph showing the ratio of NREM sleep stage 3 (N3) to total sleep time in the control food group and the test food group.
Fig. 7 is a graph showing the amount of change in the ratio of NREM sleep stage to total sleep time in the control food group and the test food group.
Fig. 8 is a graph showing the number of wake after sleep onset in the control food group and the test food group.
Fig. 9 is a graph showing the amount of change in the number of wake after sleep onset in the control food group and the test food group.
Fig. 10 is a graph showing the number of wake after sleep onset in two hours before waking up in the control food group and the test food group.
Fig. 11 is a graph showing the amount of change in the number of wake after sleep onset in two hours before waking up in the control food group and the test food group.
Fig. 12 is a graph showing NREM sleep latency in the control food group and the test food group.
Fig. 13 is a graph showing the amount of change in NREM sleep latency in the control food group and the test food group.
Fig. 14 is a graph showing δ power in the first sleep cycle in the control food group and the test food group.
Fig. 15 is a graph showing the amount of change in δ power in the first sleep cycle in the control food group and the test food group.
Fig. 16 is a graph showing sleep efficiency in the control food group and the test food group.
Fig. 17 is a graph showing the amount of change in sleep efficiency in the control food group and the test food group.
Fig. 18 is a graph showing sleep duration in the control food group and the test food group.
Fig. 19 is a graph showing the amount of change in sleep duration in the control food group and the test food group.
Fig. 20 is a graph showing the L-ergothioneine concentration in plasma in the control food group and the test food group.

### DESCRIPTION OF EMBODIMENTS

The composition for sleep improvement of the present invention contains L-ergothioneine or a salt thereof as an active ingredient.

In the present invention, the term "sleep improvement" refers to improving sleep quality and/or increasing sleep duration. The composition for sleep improvement of the present invention, which contains L-ergothioneine or a salt thereof as an active ingredient, can improve sleep quality. In one embodiment, L-ergothioneine or a salt thereof is effective in increasing sleep duration. The composition for sleep improvement of the present invention can be used for improving sleep quality and/or increasing sleep duration. In one embodiment, the composition for sleep improvement of the present invention is preferably used for improving sleep quality. L-ergothioneine or a salt thereof is present in natural products and food and beverages, and it is a compound that has been used as a food ingredient. Thus, continuous ingestion of L-ergothioneine or a salt thereof, for example, is less likely to cause problems in terms of safety.

The salt of L-ergothioneine is not limited as long as it is a pharmacologically acceptable salt or a dietary acceptable salt, and it may be either an acid salt or a basic salt. Examples of the acid salt include inorganic acid salts such as hydrochloride, sulfate, nitrate, and phosphate, and organic acid salts such as acetate, citrate, maleate, malate, oxalate, lactate, succinate, fumarate, and propionate. Examples of the basic salt include alkali metal salts such as sodium salt and potassium salt, and alkaline earth metal salts such as calcium salt and magnesium salt.

L-ergothioneine or a salt thereof that can be used may be a chemically synthesized product or a purified extract of a natural product. L-ergothioneine is abundantly present in golden/yellow oyster mushrooms (binomial name: *Pleurotus cornucopiae* var. *citrinopileatus*) belonging to the genus *Pleurotus* of the family Pleurotaceae. L-ergothioneine is also present in mushrooms such as common mushrooms (binomial name: *Agaricus bisporus*) including white mushroom, cremini mushroom, and portabella mushroom; grey oyster mushroom (binomial name: *Pleurotus ostreatus*), shiitake (binomial name: *Lentinula* edodes), hen-of-the-wood (binomial name: *Grifola Frondosa*), reishi mushuroom (binomial name: *Ganoderma lucidum*), lion's mane mushroom (binomial name: *Hericium erinaceus*), *Yanagi-matsutake* (binomial name: *Agrocybe aegerita*), girolle (binomial name: *Cantharellus cibarius*), porcini (binomial name: *Boletus edulis*), and common morel (binomial name: *Morchella esculenta*). When L-ergothioneine is obtained from a natural product, preferably, it is extracted from a golden/yellow oyster mushroom, for example. L-ergothioneine or a salt thereof can also be produced by microbial fermentation. An extract containing L-ergothioneine or a salt thereof produced by microbial fermentation or a purified product thereof may be used. L-ergothioneine can be extracted and purified from natural products by known methods. L-ergothioneine or a salt thereof may be in an isolated form. In the present invention, a mushroom extract containing L-ergothioneine or a salt thereof, for example, may be added to the composition of the present invention, as long as the effect of the present invention is obtained.

In the present invention, examples of the sleep quality improvement include reducing sleep latency (time from wakefulness to falling asleep), reducing the number and duration of wake after sleep onset, and increasing the ratio of non-rapid eye movement sleep (NREM sleep) duration to total sleep time. In the present invention, for example, the sleep latency, the number and duration of wake after sleep onset, or the ratio of NREM sleep duration to total sleep time can be used as an index for evaluation of sleep quality. NREM sleep latency (latency until the first episode of NREM sleep) can also be used as an index for evaluation of sleep quality. In the case of humans, the NREM sleep duration or sleep latency may be evaluated, for example, by a non-invasive measurement method using an electroencephalograph or the like. The sleep improving effect of the present invention can be evaluated, for example, by orally feeding or orally administering a human or animal subject (e.g., a mouse or rat) with the composition for sleep improvement of the present invention and examining sleep quality or sleep duration after the oral feeding or oral administration by the above-described method or the like.

As shown in Examples described later, stress-induced rats showed a lower sleep quality, a longer sleep latency, increased number and duration of wake after sleep onset, and a reduced NREM sleep duration relative to total sleep time (longer rapid eye movement sleep (REM sleep) time). Rats orally fed with L-ergothioneine showed a reduction in sleep latency which had been increased by stress induction. Ingestion of L-ergothioneine also reduced the number and duration of wake after sleep onset. L-ergothioneine increased the ratio of NREM sleep duration to total sleep time.

In Examples described later, subjects were fed with a food containing L-ergothioneine, and their brain waves during sleep were measured to evaluate sleep quality and sleep duration. According to the results, the ratio of NREM sleep duration to total sleep time was increased by ingestion of L-ergothioneine, compared to non-ingestion. Ingestion of L-ergothioneine also reduced the number and duration of wake after sleep onset. L-ergothioneine acted to reduce NREM sleep latency so as to reduce sleep latency. Further, ingestion of L-ergothioneine resulted in an increased ratio of deep sleep with a tendency of an increase in ratio of NREM sleep stage 3 and a tendency of an increase in δ power in the first sleep cycle. Ingestion of L-ergothioneine also improved sleep efficiency, for example. Ingestion of L-ergothioneine also increased sleep duration.

Thus, L-ergothioneine or a salt thereof acts to improve sleep, and can be used as an active ingredient of a composition for sleep improvement.

In one embodiment, the composition for sleep improvement of the present invention can be used for at least one sleep improvement selected from the group consisting of reduction in sleep latency, reduction in duration and number of wake after sleep onset, increase in ratio of NREM sleep duration to total sleep time, and increase in sleep duration. In one embodiment, the composition for sleep improvement of the present invention is preferably used for at least one sleep improvement selected from the group consisting of reduction in sleep latency, reduction in duration and number of wake after sleep onset, and increase in ratio of NREM sleep duration to total sleep time. L-ergothioneine or a salt thereof can be used for improvement of poor sleep quality caused by stress, for example, for at least one sleep improvement selected from the group consisting of reduction in sleep latency increased by stress, reduction in number and duration of wake after sleep onset which are increased by stress, and increase in ratio of NREM sleep duration to total sleep time which is reduced by stress. NREM sleep can be divided, for example, into three stages 1 to 3 according to sleep depth. Stage 3 is deep sleep. In the present invention, the increase in ratio of NREM sleep duration to total sleep time may be an increase in ratio of total NREM sleep duration to total sleep time and/or an increase in ratio of NREM sleep stage 3 to total sleep time. In one embodiment, the increase in ratio of NREM sleep duration to total sleep time is preferably an increase in ratio of NREM sleep stage 3 to total sleep time. The NREM sleep stages 1 to 3 can be determined by brain waves during sleep. In the present invention, the NREM sleep stages 1, 2, and 3 refer to NREM sleep stages 1, 2, and 3 classified according to sleep depth by measuring brain waves using an electroencephalograph "SLEEPSCOPE" (product name) available from SleepWell Co., Ltd. or by electroencephalography comparable to a method that uses the electroencephalograph. The composition for sleep improvement of the present invention shows an excellent effect of resolving dissatisfaction in sleep, such as having trouble with falling asleep or shallow sleep (e.g., improving falling asleep or alleviating deep sleep failure (nonrestorative sleep)).

The composition for sleep improvement of the present invention can be used for, for example, preventing or alleviating a condition or disease such as a sleep disorder. The sleep disorder may be a stress-induced sleep disorder. The sleep disorder includes dyssomnia such as insomnia. Insomnia refers to a condition with one of the following symptoms: difficulty of initiating sleep (difficulty falling asleep), wake after sleep onset, early morning awakening, and deep sleep failure. Insomnia may be stress-induced insomnia. In one embodiment, the composition for sleep improvement of the present invention can be used for alleviating at least one insomnia condition selected from the group consisting of difficulty of initiating sleep, wake after sleep onset, early morning awakening, and deep sleep failure. In one embodiment, the composition for sleep improvement of the present invention can be suitably used for reducing wake after sleep onset, especially reducing the duration and number of wake after sleep onset.

Preventing a condition or disease encompasses preventing the onset of a condition or disease, delaying the onset of a condition or disease, reducing the incidence of a condition or disease, reducing the onset risk of a condition or disease, and the like. Alleviating a condition or disease encompasses helping a subject recover from a condition or disease, alleviating a symptom of a condition or disease, favorably changing a symptom of a condition or disease, delaying or preventing the progress of a condition or disease, and the like.

The composition for sleep improvement of the present invention is applicable for therapeutic use (medical use) or non-therapeutic use (non-medical use). The "non-therapeutic" is a concept that does not include medical activities, i.e., a concept that does not include methods of surgery, therapy, or diagnosis of humans.

The composition for sleep improvement of the present invention can be provided in the form of a food or beverage, a pharmaceutical product, a quasi-pharmaceutical product, feed, or the like, for example. The composition for sleep improvement of the present invention may be a food or beverage, a pharmaceutical product, a quasi-pharmaceutical product, feed, or the like for sleep improvement by itself, or may be a material, a preparation, or the like that is added thereto.

The composition for sleep improvement of the present invention may be provided as an agent or the like, for example, but it is not limited thereto. The agent can be directly provided as a composition, or can be provided as a composition containing the agent. In one embodiment, the composition for sleep improvement of the present invention can also be referred to as a "sleep improver".

In order to sufficiently obtain the effect of the present invention, preferably, the composition for sleep improvement of the present invention is an oral composition. The oral composition may be a food or beverage, an oral pharmaceutical product, an oral quasi-pharmaceutical product, or feed, preferably a food or beverage or an oral pharmaceutical product, still more preferably a food or beverage.

The composition for sleep improvement of the present invention may contain optional additives and optional components in addition to L-ergothioneine or a salt thereof, as long as the effect of the present invention is not impaired. Such additives and components can be selected according to the composition form or the like. Those that can be generally used in foods, beverages, pharmaceutical products, quasi-pharmaceutical products, feed, and the like can be used. When the composition for sleep improvement is provided as a food or beverage, a pharmaceutical product, a quasi-pharmaceutical product, feed, or the like, any production method may be used, and such a product can be produced by a common method.

For example, when the composition for sleep improvement of the present invention is provided as a food or beverage, various types of food or beverages can be provided by adding a component usable in food or beverages (e.g., a food material or an optional food additive) to L-ergothioneine or a salt thereof. Non-limiting examples of the food or beverages include general foods and beverages, health foods, health beverages, foods with function claims, foods for specified health uses, dietary supplements, and foods and beverages for the sick. The health foods, foods with function claims, foods for specified health uses, dietary supplements, and the like can be used, for example, in various forms of preparations such as fine granules, tablets, granules, powders, capsules, chewable tablets, syrups, liquids, and liquid foods.

When the composition for sleep improvement of the present invention is provided as a pharmaceutical product or a quasi-pharmaceutical product, for example, a pharmacologically acceptable carrier, an optional additive, or the like can be added to L-ergothioneine or a salt thereof to provide pharmaceutical products or quasi-pharmaceutical products in various dosage forms. Such a carrier, an additive, or the like may be any pharmacologically acceptable one that can be used in pharmaceutical products or quasi-pharmaceutical products. Examples thereof include excipients, binders, disintegrants, lubricants, antioxidants, and colorants. One or more of these can be used. The form of administration of the pharmaceutical product or the quasi-pharmaceutical product may be an oral or parenteral. Oral administration is preferred in order to obtain the effect of the present invention more sufficiently. When the composition for sleep improvement of the present invention is provided as a pharmaceutical product or a quasi-pharmaceutical product, preferably, it is an oral pharmaceutical product or an oral quasi-pharmaceutical product. Examples of dosage forms for oral administration include liquids, tablets, powders, fine granules, granules, sugar-coated tablets, capsules, suspensions, emulsions, and chewable tablets. Examples of dosage forms for parenteral administration include injection and infusion. The pharmaceutical product and the quasi-pharmaceutical product may be for non-human animals.

When the composition for sleep improvement of the present invention is provided as feed, L-ergothioneine or a salt thereof is simply added to feed. The feed includes feed additives. Examples of the feed include livestock feed for animals such as cows, pigs, chickens, sheep, and horses; feed for small animals such as rabbits, rats, and mice; and pet food for animals such as dogs, cats, and birds.

The amount of L-ergothioneine or a salt thereof in the composition for sleep improvement of the present invention is not limited, and can be set according to the composition form and the like.

The amount of L-ergothioneine or a salt thereof, for example, in terms of L-ergothioneine, in the composition for sleep improvement of the present invention is preferably 0.0001 wt% or more, more preferably 0.001 wt% or more, and is preferably 90 wt% or less, more preferably 50 wt% or less. In one embodiment, the amount of L-ergothioneine or a salt thereof, in terms of L-ergothioneine in the composition for sleep improvement is preferably 0.0001 to 90 wt%, more preferably 0.001 to 50 wt%. In one embodiment, when the composition for sleep improvement of the present invention is provided as a food or beverage, a pharmaceutical product, a quasi-pharmaceutical product, feed, or the like, preferably, the amount of L-ergothioneine or a salt thereof is in the above ranges.

The composition for sleep improvement of the present invention can be ingested or administered by a method appropriate to the composition form. In order to sufficiently obtain the effect of the present invention, preferably, the composition for sleep improvement of the present invention is orally ingested (orally administered) . The intake (dosage) of the composition for sleep improvement of the present invention is not limited, as long as it is an amount that achieves the sleep improving effect (in one embodiment, preferably, a sleep quality improving effect). The intake may be appropriately set according to the administration form, administration method, body weight of a subject, and the like.

In one embodiment, when a human (adult) is subjected to oral ingestion or administration of the composition for sleep improvement of the present invention, the intake of L-ergothioneine or a salt thereof per 60 kg of body weight per day in terms of L-ergothioneine is preferably 1 mg or more, more preferably 5 mg or more, still more preferably 10 mg or more, and is preferably 500 mg or less, more preferably 200 mg or less, still more preferably 100 mg or less, particularly preferably 50 mg or less. In one embodiment, when a human (adult) is subjected to oral ingestion or administration of the composition, the intake of L-ergothioneine or a salt thereof per 60 kg of body weight per day in terms of L-ergothioneine can be less than 50 mg or can be 45 mg or less. In one embodiment, when a human (adult) is subjected to oral ingestion or administration, the intake of L-ergothioneine or a salt thereof per 60 kg of body weight per day in terms of L-ergothioneine is preferably 1 to 500 mg, more preferably 5 to 200 mg, still more preferably 10 to 100 mg, particularly preferably 10 to 50 mg. The intake of L-ergothioneine or a salt thereof per 60 kg of body weight per day in terms of L-ergothioneine may be 10 mg or more and less than 50 mg and may be 10 to 45 mg. Preferably, the above amount of the composition is ingested or administered in one or more portions per day, for example, in one portion or several portions (for example, two or three portions) per day. In one embodiment of the present invention, the composition for sleep improvement may be an oral composition for feeding or administering a human with the above amount of L-ergothioneine or a salt thereof per 60 kg of body weight per day.

In one embodiment, when the composition for sleep improvement of the present invention is parenterally administered to a human (adult), the dosage of L-ergothioneine or a salt thereof per 60 kg body weight per day in terms of L-ergothioneine is, for example, preferably 1 to 500 mg, more preferably 5 to 200 mg, still more preferably 10 to 100 mg, particularly preferably 10 to 50 mg. When the composition is parenterally administered to a human (adult), the dosage of L-ergothioneine or a salt thereof per 60 kg body weight per day in terms of L-ergothioneine may be 10 mg or more and less than 50 mg or may be 10 to 45 mg.

### Continuous ingestion or administration of

L-ergothioneine or a salt thereof is likely to result in a better sleep improving effect. Thus, in a preferred embodiment, the composition for sleep improvement of the present invention is continuously ingested or administered. In one embodiment of the present invention, the composition for sleep improvement is continuously ingested or administered preferably for at least one week, more preferably for at least two weeks.

A subject (administration subject) to be fed or administered with the composition for sleep improvement of the present invention is not limited. The subject is preferably a human or non-human mammal, more preferably a human.

The subject to be fed or administered with the composition for sleep improvement of the present invention may be a subject who needs or wants to improve sleep, for example, a subject who needs or wants to improve sleep quality or a subject who needs or wants to increase sleep duration. The administration subject may be, for example, a human with poor sleep quality (preferably, a human with poor sleep quality caused by stress) or a healthy person who needs or wants to have higher quality sleep. In one embodiment, the administration subject may be a subject with a sleep disorder (preferably, a human with a sleep disorder) or a subject with insomnia (preferably, a human with insomnia) . The administration subject may also be, for example, a human with at least one insomnia condition selected from the group consisting of difficulty of initiating sleep, wake after sleep onset, early morning awakening, and deep sleep failure, or a human who needs or wants to improve such a condition.

The composition for sleep improvement of the present invention may be labeled with function claims based on sleep improvement. The composition for sleep improvement of the present invention may be labeled with one or more function claims such as "reducing drowsiness upon awakening", "improving sleep quality", "improving falling asleep", "improving deep sleep", "sense of satisfaction upon awakening", and/or "recovery from fatigue".

In one embodiment of the present invention, preferably, the composition for sleep improvement of the present invention is a food or beverage labeled with one or more of the above function claims. The labels may be labels indicating use for obtaining these functions.

The present invention also encompasses the following method and use:
a method of improving sleep, the method including feeding or administering L-ergothioneine or a salt thereof; and
use of L-ergothioneine or a salt thereof for improving sleep.

The method and use may be therapeutic or non-therapeutic. Feeding or administering a subject with L-ergothioneine or a salt thereof can improve sleep quality of the subject. In one embodiment, ingestion or administration of L-ergothioneine or a salt thereof can increase sleep duration. Oral ingestion or administration of L-ergothioneine or a salt thereof is preferred. The above method may be a method of reducing sleep latency, reducing the duration and number of wake after sleep onset, and/or increasing the ratio of NREM sleep duration to total sleep time. The use may be use for at least one selected from the group consisting of reduction in sleep latency, reduction in duration and number of wake after sleep onset, and increase in ratio of NREM sleep duration to total sleep time.

In the use and the method, L-ergothioneine or a salt thereof, preferred embodiments thereof, and the like are as described above for the composition for sleep improvement of the present invention. Preferably, the method and the use include feeding or administering a subject with L-ergothioneine or a salt thereof one or more times per day, for example, one to several times (e.g., two to three times) per day. The uses are preferably for a human or non-human mammal, more preferably for a human. In one embodiment, L-ergothioneine or a salt thereof can be used, for example, for preventing or alleviating sleep disorder. L-ergothioneine or a salt thereof can be used for preventing or alleviating insomnia. L-ergothioneine or a salt thereof can be used for, for example, alleviating at least one insomnia condition selected from the group consisting of difficulty of initiating sleep, wake after sleep onset, early morning awakening, and deep sleep failure. The present invention also encompasses a method of alleviating at least one insomnia condition selected from the group consisting of difficulty of initiating sleep, wake after sleep onset, early morning awakening, and deep sleep failure, the method including feeding or administrating L-ergothioneine or a salt thereof.

In the method and the use, L-ergothioneine or a salt thereof is simply used in an amount (effective amount) that provides a sleep improving effect. A preferred dosage of L-ergothioneine or a salt thereof, a preferred administration subject, and the like are as described above for the composition for sleep improvement of the present invention. L-ergothioneine or a salt thereof may be directly ingested or administered, or may be ingested or administered as a composition containing L-ergothioneine or a salt thereof. For example, the composition for sleep improvement of the present invention may be ingested or administered.

L-ergothioneine or a salt thereof can also be used for producing a food or beverage, a pharmaceutical product, a quasi-pharmaceutical product, feed, or the like used for improving sleep. In one embodiment, the present invention also encompasses use of L-ergothioneine or a salt thereof for producing a composition for sleep improvement.
The present invention also encompasses L-ergothioneine or a salt thereof for use in improvement of sleep;
L-ergothioneine or a salt thereof for use in prevention or alleviation of a sleep disorder; L-ergothioneine or a salt thereof for use in prevention or alleviation of insomnia;
L-ergothioneine or a salt thereof for use in alleviation of at least one insomnia condition selected from the group consisting of difficulty of initiating sleep, wake after sleep onset, early morning awakening, or deep sleep failure; a composition containing L-ergothioneine or a salt thereof for use in improvement of sleep; a composition containing L-ergothioneine or a salt thereof for use in prevention or alleviation of a sleep disorder; a composition containing L-ergothioneine or a salt thereof for use in prevention or alleviation of insomnia; and a composition containing L-ergothioneine or a salt thereof for use in alleviation of at least one insomnia condition selected from the group consisting of difficulty of initiating sleep, wake after sleep onset, early morning awakening, or deep sleep failure. The composition may be the above-described composition for sleep improvement, and may be a food or beverage, a pharmaceutical product, a quasi-pharmaceutical product, feed, or the like.

### EXAMPLES

The present invention is described in further detail below with reference to examples, but the scope of the present invention is not limited thereto.

A series of animal experiments was performed based on a plan approved by the relevant chief through evaluation of the in-house animal experiment committee, in compliance with the animal welfare management laws and other related laws and regulations.

### <Example 1>

### (Stress sleep disorder model)

The following method was used to induce stress in rats in an ergothioneine administration group (N = 5) and a non-administration group (N = 7). During the test period, the rats were kept under a 12-hour light-dark cycle.

A Sprague-Dawley (SD) rat (8 weeks of age) was introduced into a cage where a larger and highly aggressive Brown-Norway (BN) rat was kept. These two rats were housed together for 10 minutes to make sure contact (direct contact) between the rats as the BN rat chasing after or hovering over the SD rat. Then, the SD rat and the BN rat were kept together for 24 hours in a breeding cage having compartments separated by a transparent perforated partition (indirect contact). Stress was induced in the SD rat in the same manner on the following day. A BM rat different from the one on the preceding day was used as a stressor. Stress was induced once a day for five consecutive days (from Monday to Friday), and then only indirect contact was allowed for two days on the weekend. This process was repeated for two cycles.

Thus, stress induction under conditions combining direct contact and indirect contact was performed 10 times in total, and stress was induced for 12 days in total.

### (Administration of L-ergothioneine)

The ergothioneine administration group was orally administered with L-ergothioneine from seven days before stress induction to the end of stress induction. L-ergothioneine was used in the form of an aqueous solution of L-ergothioneine dissolved in water (L-ergothioneine concentration: 0.25 mg/mL) . The dosage of L-ergothioneine was about 20 mg per 1 kg body weight per day. The non-administration group was orally administered with water instead of the aqueous L-ergothioneine solution.

The control (stress (-)) group (N = 4) was orally administered with water without the stress induction.

### (Measurement of brain waves during sleep)

Electrodes were implanted in the rats to record brain waves, and brain waves during sleep and wakefulness were recorded for 24 hours after the end of stress induction. NREM sleep, REM sleep, and awake state were determined from the brain waves using biological signal analysis software Spike2 (available from CED), and the duration of episode of each of these states was calculated. Tukey's multiple comparisons test (*: p < 0.05, **: p < 0.005, ****: p < 0.0001) was performed for significance testing.

### (Results)

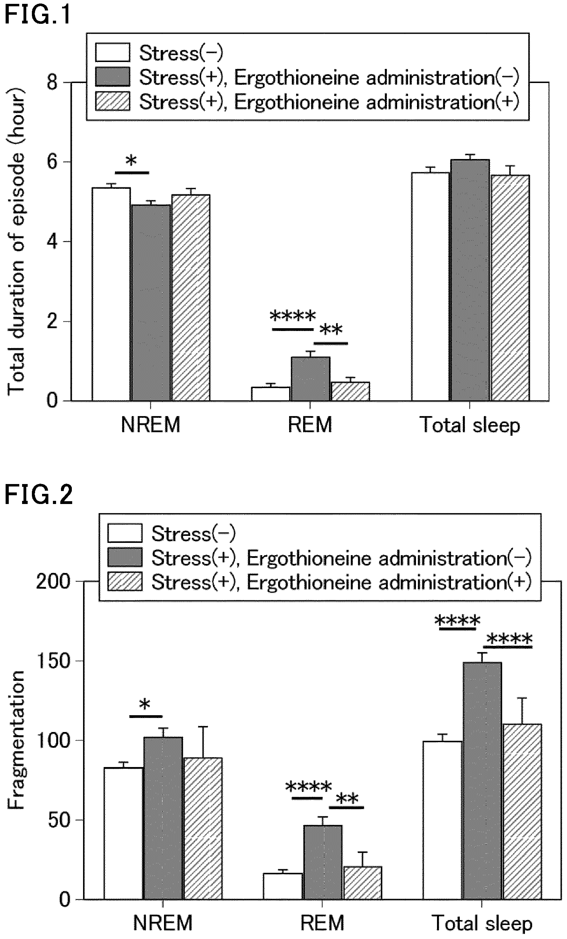

Fig. 1, Fig. 2, and Fig. 3 show the results. In Fig. 1 to Fig. 3, "Stress (-)" represents a control group (a group without stress induction), "Stress (+), ergothioneine administration (-)" represents a non-administration group (a group with stress induction and without administration of L-ergothioneine), and "Stress (+), ergothioneine administration (+)" represents an ergothioneine administration group (a group with stress induction and administration of L-ergothioneine). The results in Fig. 1 to Fig. 3 are presented in means ± standard error for each group. The white bars represent the control group. The black bars represent the non-administration group. The hatched bars represent the ergothioneine administration group.

Fig. 1 shows the duration of episode of each sleep stage (NREM sleep or REM sleep) and sleep duration in each group during the light phase. In the light phase, the stress induction reduced the duration of episode of NREM sleep (NREM), but increased the duration of episode of REM sleep (REM), as compared to the control group. Administration of L-ergothioneine improved these phenomena to the level of the control group without stress induction (Fig. 1). Although the ratio of NREM sleep duration to total sleep time was reduced by stress, administration of L-ergothioneine increased the ratio of NREM sleep duration to total sleep time. "Sleep duration" refers to the actual sleep time, excluding wake time during sleep. "Total sleep time" refers to the time including sleep duration and wake time during sleep. Sleep duration in rats can be regarded as equivalent to total sleep time in humans .

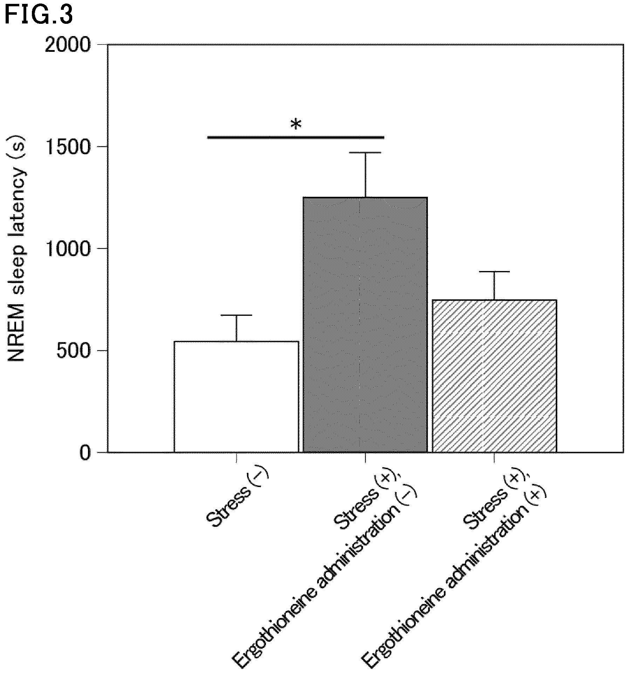

Fig. 2 shows the number of sleep fragmentation episodes in each sleep stage (the number of fragmentation episodes in NREM sleep or REM sleep) and the total number of sleep fragmentation episodes during the light phase. "Total sleep" in Fig. 2 represents the total number of sleep fragmentation episodes. Fragmentation in REM sleep was increased in the non-administration group with stress induction, compared to the control group. The ergothioneine administration group showed reduced fragmentation to a level comparable to that of the control group (Fig. 2). Fig. 3 shows the latency (sec) from the start of the light phase to the first episode of NREM sleep in each group. NREM sleep latency in rats can be regarded as equivalent to sleep latency. The non-administration group showed an increased sleep latency, compared to the control group. Administration of L-ergothioneine exhibited an action to reduce an increase in sleep latency (Fig. 3).

Administration of L-ergothioneine reduced sleep latency, which had been increased by stress induction, to a level comparable to that of control group without stress induction.

### <Example 2>

### (Sleep quality evaluation test for humans)

In order to examine a sleep improving effect such as a sleep quality-improving effect of a supplement containing ergothioneine, male and female adults feeling stressed on a daily basis and dissatisfied with their sleep (test food group: 46 subjects; control food group: 46 subjects; 92 subjects in total) were subjected to a placebo-controlled, randomized, double-blind, parallel-group comparative study in which one capsule containing 20 mg of L-ergothioneine (test food) or one capsule not containing L-ergothioneine (control food) was ingested daily for four weeks. The sleep quality and sleep duration were evaluated by measuring brain waves during sleep using an electroencephalograph "SLEEPSCOPE" (product name) available from SleepWell Co., Ltd. A 2-sample t-test (**: p < 0.01, *: p < 0.05, †: p < 0.1, vs. control food group) was performed for significance testing between the groups.

### (Types of food for evaluation)

Two types of food indistinguishable in terms of appearance, flavor, and the like were used for evaluation.

### - Test food: capsule containing a test substance (L-ergothioneine 20 mg)

### - Control food: capsule not containing a test substance

Each type of food for evaluation contained raw materials such as dextrin, hydroxypropyl cellulose, carrageenan, potassium chloride, and titanium oxide, in addition to the test substance. The control food was produced using the same raw materials used in the test food, except that the test substance (L-ergothioneine) was not added.

Brain waves during sleep were measured before the start of ingestion of the food for evaluation (pre-test) and after four weeks of ingestion of the food. The pre-test measurement of brain waves was regarded as "pre-examination", and the measurement of brain waves after four weeks of ingestion of the food for evaluation was regarded as "week 4 examination". The L-ergothioneine concentration (µM) in blood of each subject was also measured at the pre-examination and at the week 4 examination.

NREM sleep is divided into three stages 1 to 3 according to sleep depth by measuring brain waves using an electroencephalograph "SLEEPSCOPE" available from SleepWell Co., Ltd.

The following items were measured and evaluated as indices of sleep quality.

Ratio (%) of each sleep stage (NREM sleep stage 1, 2, or 3) to total sleep time; number of wake after sleep onset (frequency); number of wake after sleep onset in two hours before waking up (frequency), NREM sleep latency (min); δ power in the first sleep cycle (delta power during NREM sleep in the first sleep cycle) (µV²); and sleep efficiency (%).

### (Test results)

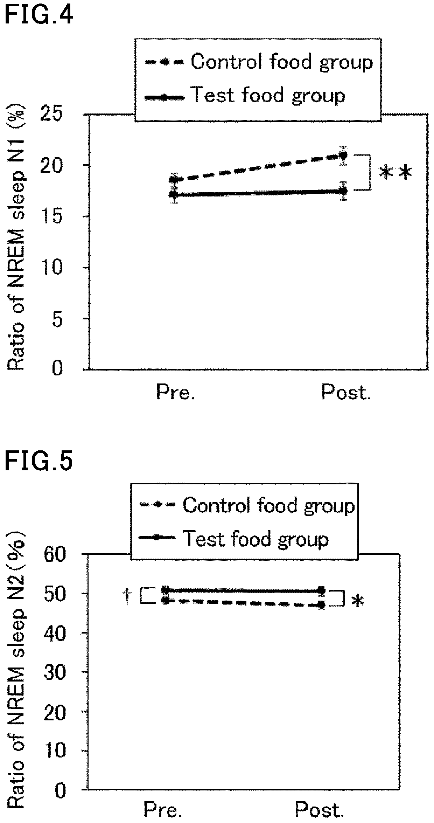

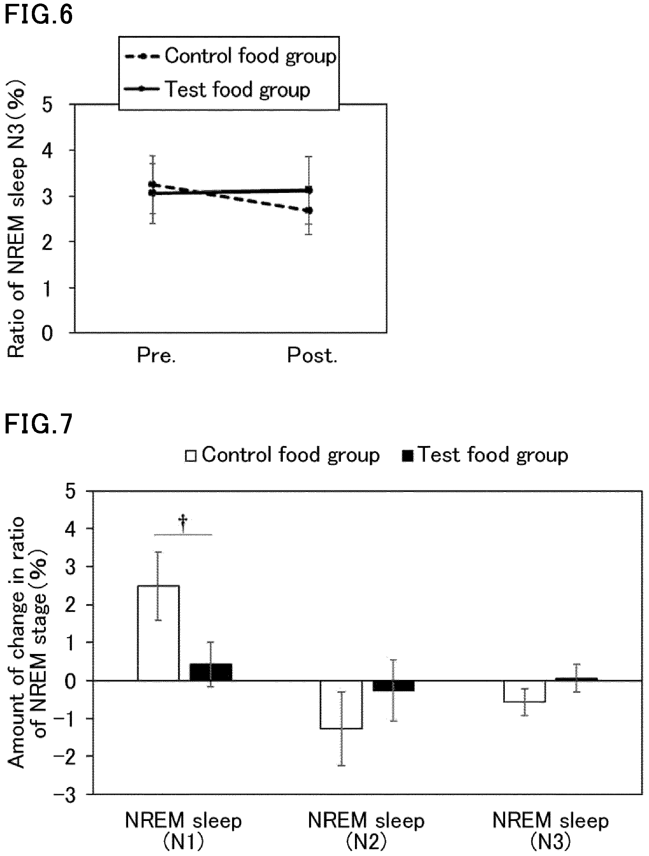

Figs. 4 to 19 show results of main evaluation items for sleep quality (ratio of total time of each sleep stage to total sleep time; number of wake after sleep onset; number of wake after sleep onset in two hours before waking up; NREM sleep latency; δ (delta) power in the first sleep cycle, and sleep efficiency) and sleep duration, as determined by measuring brain waves. Figs. 4 to 19 show the measured values and the amount of change between measurements at the pre-examination and at the week 4 examination (**: p < 0.01, *: p < 0.05, †: p < 0.1, vs. control food group). The amount of change is a value obtained by subtracting the measured value at the pre-examination from the measured value at the week 4 examination ("measured value at week 4 examination" - "measured value at pre-examination") . The results shown in Fig. 4 to Fig 20 are presented in means ± standard error for each group. A 2-sample t-test (**: p < 0.01, *: p < 0.05, †: p < 0.1, vs. control food group) was performed for significance testing between the groups. In Fig. 4, Fig. 5, Fig. 6, Fig. 8, Fig. 10, Fig. 12, Fig. 14, Fig. 16, Fig. 18, and Fig. 20, "Pre." refers to the pre-examination, and "Post." refers to the week 4 examination. The solid lines represent the test food group, and the dashed lines represent the control food group. In Fig. 7, Fig. 9, Fig. 11, Fig. 13, Fig. 15, Fig. 17, and Fig. 19, the black bars represent the test food group, and the white bars represent the control food group.

Fig. 4, Fig. 5, and Fig. 6 each show the measured ratio of NREM sleep stage to total sleep time in each group. Fig. 7 shows the amount of change in the ratio of each NREM sleep stage to total sleep time in each group.

Fig. 4 is a graph showing the ratio of NREM sleep stage 1 (N1) to total sleep time. Fig. 5 is a graph showing the ratio of NREM sleep stage 2 (N2) to total sleep time. Fig. 6 is a graph showing the ratio of NREM sleep stage 3 (N3) to total sleep time. Fig. 7 is a graph showing the amount of change in the ratio of NREM sleep stage to total sleep time.

The control food group showed an increased ratio of NREM sleep stage 1 serving as an index of shallow sleep, and reduced ratios of NREM sleep stages 2 and 3 serving as indices of deep sleep. The ratio of NREM sleep stage 1 which was increased in the control food group was significantly suppressed by the intervention of L-ergothioneine. The ratios of NREM sleep stages 2 and 3 which were reduced in the control food group were increased by the intervention of L-ergothioneine. Generally, the intervention of L-ergothioneine increased the ratio of deep sleep. The test food group showed an increased total time of NREM sleep stages 1 to 3 relative to total sleep time, compared to the control food group. L-ergothioneine increased the ratio of NREM sleep duration to total sleep time.

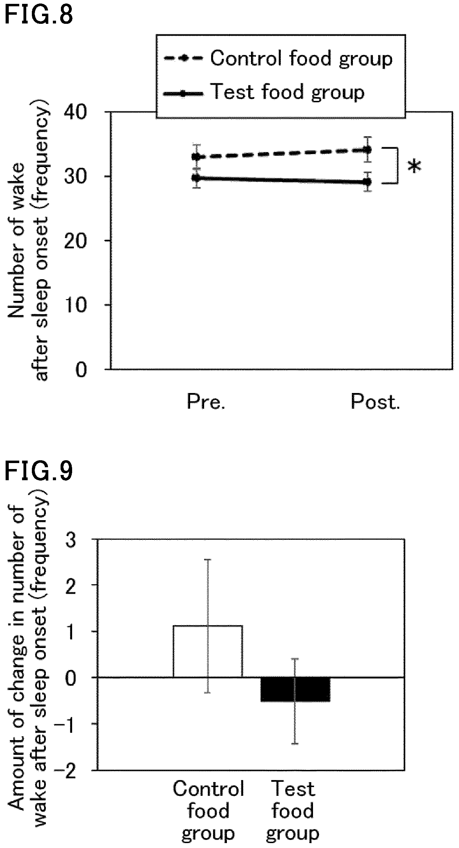

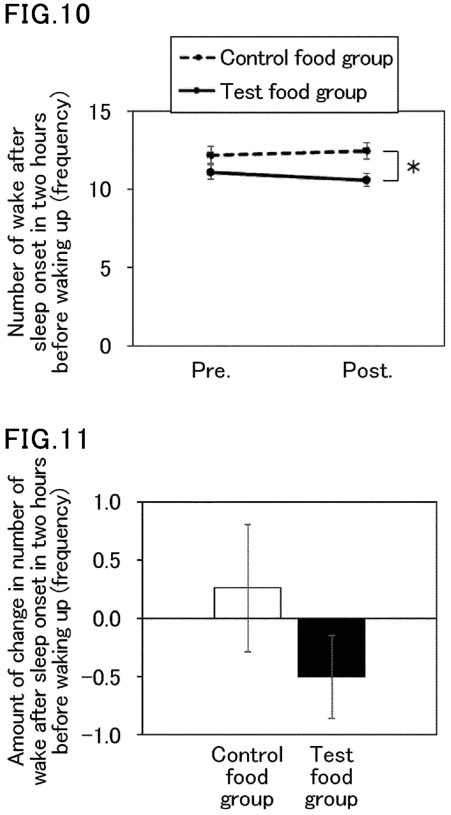

Fig. 8 shows the measured number of wake after sleep onset in each group. Fig. 9 shows the amount of change in the number of wake after sleep onset in each group. Fig. 10 shows the measured number of wake after sleep onset in two hours before waking up in each group. Fig. 11 shows the amount of change in the number of wake after sleep onset in two hours before waking up in each group. The number of wake after sleep onset in two hours before waking up is used as an index of early morning awakening, and is known that a smaller number leads to easier waking up. While the control food group showed an increased number of wake after sleep onset, and an increased number of wake after sleep onset in two hours before waking up, which resulted in an increased sleep fragmentation, the intervention of L-ergothioneine significantly suppressed sleep fragmentation. L-ergothioneine reduced the number and duration of wake after sleep onset.

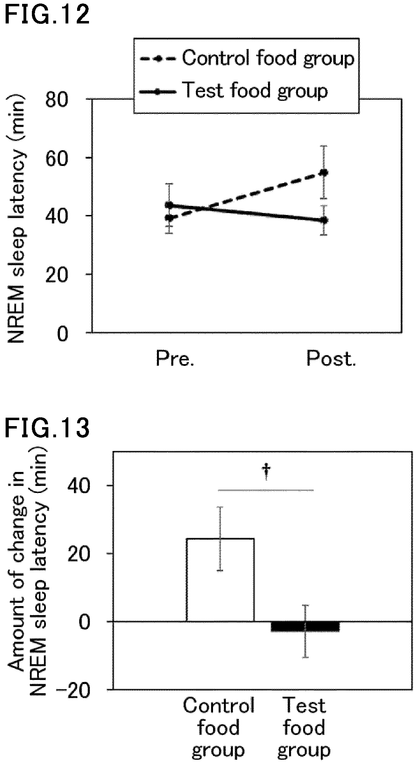

Fig. 12 shows the measured NREM sleep latency in each group . Fig. 13 shows the amount of change in NREM sleep latency in each group. While the control food group showed an increased time to reach deep sleep, the intervention of L-ergothioneine reduced an increase in time to reach deep sleep. The test food group fed with L-ergothioneine showed a reduced sleep latency, compared to the control food group. L-ergothioneine reduced NREM sleep latency, which reduced sleep latency.

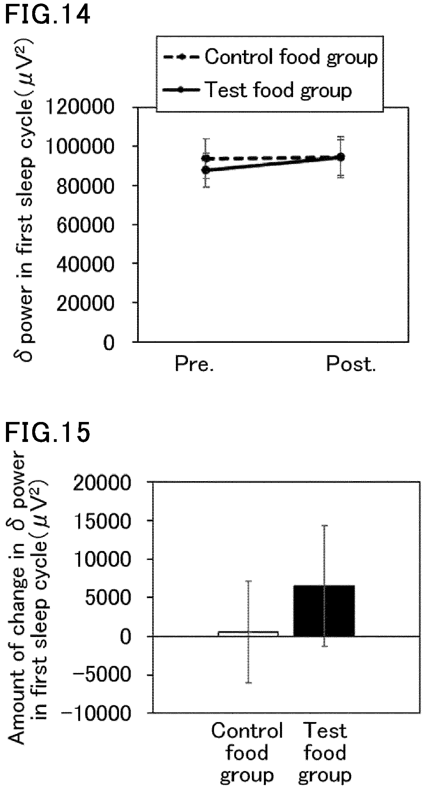

Fig. 14 shows the measured δ power in the first sleep cycle in each group. Fig. 15 shows the amount of change in δ power in the first sleep cycle in each group. The δ power in the first sleep cycle is known as an index of sleep depth. While the control food group showed hardly any change in δ power in the first sleep cycle, the intervention of L-ergothioneine increased δ power in the first sleep cycle. Based on the above and the results shown in Fig. 4, Fig. 5, Fig. 6, and Fig. 7, L-ergothioneine is expected to have a deep sleep inducing effect.

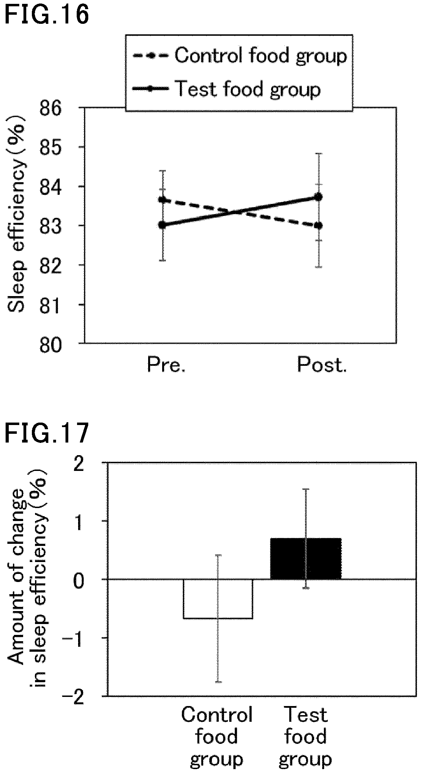

Fig. 16 shows the measured sleep efficiency in each group. Fig. 17 shows the amount of change in sleep efficiency in each group. Sleep efficiency indicates the ratio of actual sleep time during sleep, and is known as an index of sleep quality as a whole. While the control food group showed a reduced sleep efficiency, the intervention of L-ergothioneine increased sleep efficiency.

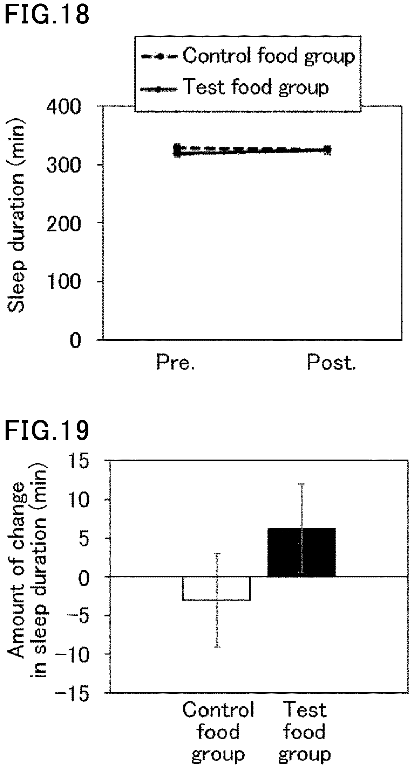

Fig. 18 shows the measured sleep duration in each group. Fig. 19 shows the amount of change in sleep duration in each group. While the control food group showed a reduced sleep duration, the intervention of L-ergothioneine increased sleep duration.

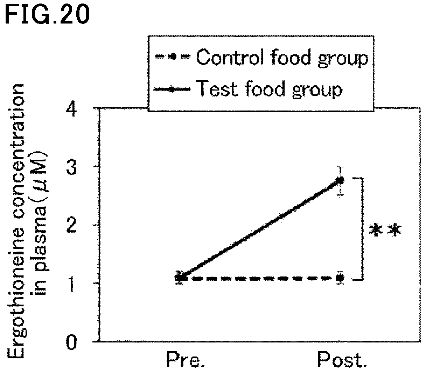

Fig. 20 shows the L-ergothioneine concentration in plasma in the control food group and the test food group. The results confirmed that the intervention of L-ergothioneine increased the L-ergothioneine concentration in plasma. The results show that an increase in L-ergothioneine concentration in blood results in a better sleep quality and a longer sleep duration.

## Claims

1. A composition for sleep improvement, the composition comprising:
L-ergothioneine or a salt thereof as an active ingredient.

2. The composition for sleep improvement according to claim 1,
wherein the sleep improvement is at least one selected from the group consisting of reduction in sleep latency, reduction in duration and number of wake after sleep onset, and increase in ratio of NREM sleep duration to total sleep time.

3. The composition for sleep improvement according to claim 1 or 2,
wherein the composition is an oral composition.

4. The composition for sleep improvement according to any one of claims 1 to 3,
wherein the composition is a food or beverage.

5. A method of improving sleep, the method comprising:
administering L-ergothioneine or a salt thereof.

6. Use of L-ergothioneine or a salt thereof for improving sleep.
